# EUROPEAN PATENT APPLICATION

(11) **EP 4 541 372 A1**
(43) Date of publication of application: **23.04.2025**
(21) Application number: 23823949.5
(22) Date of filing: 14.06.2023
(51) Int. Cl.: A61K 45/00, A61K 45/06, A61P 43/00, A61K 31/166, A61K 31/198, A61K 31/277, A61K 31/352, A61K 31/38, A61K 31/381, A61K 31/40, A61K 31/407, A61K 31/438, A61K 31/451, A61K 31/4515, A61K 31/454, A61K 31/4545, A61K 31/473, A61K 31/4743, A61K 31/48

(54) **DRUG THERAPY FOR OBSESSIVE-COMPULSIVE DISORDER TARGETING DOPAMINE D1 SIGNAL IN STRIATAL STRIOSOMES**

(30) Priority: 15.06.2022 JP 2022096675; 27.12.2022 WO PCT/JP2022/048379
(71) Applicant: Matsumoto, Shinichi, Nishinomiya-shi, Hyogo 663-8107 (JP); Goto, Satoshi, Kumamoto-shi, Kumamoto 862-0957 (JP); Shimazu, Hideki, Nishinomiya-shi, Hyogo 663-8023 (JP)
(72) Inventor: Matsumoto, Shinichi, Nishinomiya-shi, Hyogo 663-8107 (JP); Goto, Satoshi, Kumamoto-shi, Kumamoto 862-0957 (JP); Shimazu, Hideki, Nishinomiya-shi, Hyogo 663-8023 (JP)
(74) Representative: Vossius & Partner Patentanwälte Rechtsanwälte mbB
(86) International application number: PCT/JP2023/022088
(87) International publication number: WO 2023/243659

(57) **Abstract**

A pharmaceutical composition for treating obsessive-compulsive disorder is provided.

The present invention relates to (1) a pharmaceutical composition comprising a dopamine D1 receptor agonist as an effective ingredient for treating obsessive-compulsive disorder, and/or (2) a pharmaceutical composition comprising a dopamine D1 and D2 receptor stimulator, which is administered in combination with a dopamine D2 receptor inhibitor for the purpose of stimulating the dopamine D1 receptor in the striatum, and/or (3) a pharmaceutical composition comprising a dopamine D2 receptor inhibitor, which is administered in combination with a dopamine D1 and D2 receptor stimulator.

## Description

### TECHNICAL FIELD

The present patent application claims the priority and benefit under the Paris Convention based on Japanese Patent Application No. 2022-096675 (filed on June 15, 2022) and PCT/JP2022/048379 (filed on December 27, 2022), which are incorporated herein by reference in its entirety.

The present invention relates to a pharmacotherapy for treating obsessive-compulsive disorder. Specifically, the present invention belongs to the field of pharmacotherapy for treating obsessive-compulsive disorder by use of dopamine D1 receptor stimulators, and/or a combination of dopamine D1 and D2 receptor stimulators and dopamine D2 receptor inhibitors with the aim of selectively stimulating dopamine D1 receptors in the striatum.

### BACKGROUND TECHNOLOGY

Obsessive-Compulsive Disorder (OCD) is a disorder that forms the core of the category of obsessive-compulsive related disorders that are characterized by obsessive symptoms such as obsessive thoughts or compulsive behaviors, including a group of disorders called "Obsessive Compulsive and Related Disorders", as stated in Guidelines for translation of disease names/terms in DSM-5 (Diagnostic and Statistical Manual of Mental Disorders, 5th Edition). The symptoms of obsessive-compulsive disorder consist of obsessions with repetitive/persistent thoughts and urges or images, as well as compulsive acts including repeated hand washing and checking, ritualistic behaviors, and repetitive mental acts such as casting a spell and counting. Patients with obsessive-compulsive disorder experience conflict and stress since they cannot control their obsessions or compulsions due to anxiety and distress although they try to control them (Non-Patent Documents 1, 2).

The prevalence of obsessive-compulsive disorder is 1-2%, and the gender ratio is almost equal. The average age of onset is around 20 years old, and men tend to develop the disease earlier (Non-Patent Documents 1, 2). Obsessive-compulsive disorder is typically treated with a combination of the oral administration therapy of a selective serotonin reuptake inhibitor (SSRI), a type of antidepressant, and the cognitive behavioral therapy (CBT) (Non-Patent Documents 2, 3). The oral administration of SSRIs is recommended to be continued for 12 weeks from the start of administration, with a maximum dose for 4 to 6 weeks (Non-Patent Documents 2, 3, 4). Currently, SSRIs are the first-choice drugs for treating obsessive-compulsive disorder, but they require higher doses and longer periods of the drugs medication than those required for depression. Long-term prognosis studies show that the patients with obsessive-compulsive disorder who respond to SSRI treatment are merely approximately half irrespective of treatment experience of 10 years or more, that their improvement rates remain at a maximum of 30 to 40%, and that they often experience relapse even after a brief period of relief (Non-Patent Document 2). In order to clinically treat obsessive-compulsive disorder, the development of a more effective pharmacotherapy as an alternative to SSRIs is desired.

### CITATION LIST

### PATENT DOCUMENTS

### NON-PATENT DOCUMENTS

Non-Patent Document 1: American Psychiatric Association: Diagnostic and statistical manual of mental disorders, 4th ed. Text Revision. APA. Washington D.C.
Non-Patent Document 2: Koran, L. M. Hanna, G.L., Hollander. E., et al: Practice guideline for the treatment of patients with obsessive-compulsive disorder. Am J Psychiatry. 164(supple); 1-56, 2007
Non-Patent Document 3: March. J., Frances. A., Kahn. D., et al Expert consensus guidelines: Treatment of obsessive-compulsive disorder. J Clin Psychiatry, 58 (suppl.4); 1-72, 1997
Non-Patent Document 4: Denys, D.: Pharmacotherapy of obsessive-compulsive disorder and obsessive-compulsive spectrum disorders. Psychiatr Clin North Am.29; 553-584, 2006
Non-Patent Document 5: Goodman WK, Price LH, Rasmussen SA et al: The Yale-Brown obsessive-compulsive scale II. Validity. Arch Gen Psychiatry 46: 1012-1016, 1989
Non-Patent Document 6: Steketee, G and Neziroglu, E:Assessment of obsessive-compulsive disorder and spectrum disorders. Bref Treat. Crisis Interv., 3: 169-185, 2003
Non-Patent Document 7: Storch EA, De Nadai AS, Conceicao do Rosario M et al Defining severity in adults with obsessive compulsive disorder. Compr Psychiatry 63: 30-35, 2015

### SUMMARY OF THE INVENTION

### PROBLEMS TO BE SOLVED BY THE INVENTION

As stated above, there are no medicines or methods that can fully treat obsessive-compulsive disorder, and any effective therapeutical methods are being sought.

### SOLUTIONS TO THE PROBLEMS

Under these circumstances, during the course of treatment of patients with involuntary movement disorders, the inventors of the present application accidentally discovered that the administration of a small amount of levodopa along with a small amount of chlorpromazine or metoclopramide facilitated the improvement in obsessive-compulsive disorder within four weeks, thereby leading to the completion of the present invention. Specifically, the present inventors discovered for the first time that, in the dual dopamine regulation therapy using L-3,4-dihydroxyphenylalanine (L-DOPA; levodopa), which is a dopamine D1 and D2 receptor stimulator, and chlorpromazine (CPZ) or metoclopramide, which is a D2 receptor blocker, a lower dose of each drug than the standard maintenance dose, and a shorter treatment period of each drug than the usual treatment period can effectively treat obsessive-compulsive disorder.

Accordingly, the present invention encompasses the following embodiments:

### <Pharmaceutical compositions>

(1) A pharmaceutical composition for treating obsessive-compulsive disorder, which comprises a dopamine D1 receptor stimulator.
(2) The pharmaceutical composition of (1), wherein the dopamine D1 receptor stimulator is selected from the group consisting of dopamine D1 receptor agonists and dopamine D1 receptor-positive allosteric modulators.
(3) The pharmaceutical composition of (2), wherein the dopamine D1 receptor stimulator is a dopamine D1 receptor agonist.
(4) The pharmaceutical composition of (3), wherein the dopamine D1 receptor agonist is selected from the group consisting of SKF81297, SKF38393, SKF83959, SKF82526 (fenoldpam), dihydrexidine, ABT-431, A-86929, A-77636, A-68930, PF-06649751 (tavapandon), and PF-06412.
(5) The pharmaceutical composition of (2), wherein the dopamine D1 receptor stimulator is a dopamine D1 receptor-positive allosteric modulator.
(6) The pharmaceutical composition of (5), wherein the dopamine D1 receptor-positive allosteric modulator is selected from the group consisting of DETQ (2-(2,6-dichlorophenyl)-1-((1S,3R)-3-(hydroxymethyl)-5-(2-hydroxypropan-2-yl)-1-methyl-3,4-dihydroisoquinolin-2(1H)-yl)ethan-1-one), mevidalen, MLS1082, MLS6585, pyrazolyl-dihydroisoquinoline, DPTQ, CID 2886111 ((N-(6-tert-butyl-3-carbamoyl-4,5,6,7-tetrahydro-1-benzothiophen-2-yl)pyridine-4-carboxamide), LY3154885, and ASP4345.
(7) A pharmaceutical composition for treating obsessive-compulsive disorder comprising a dopamine D1 and D2 receptor agonist, wherein the composition is administered in combination with a dopamine D2 receptor inhibitor.
(8) The pharmaceutical composition of (7), wherein the dopamine D2 receptor inhibitor is selected from the group consisting of dopamine D2 receptor antagonists and dopamine D2 receptor-negative allosteric modulators.
(9) The pharmaceutical composition of (8), wherein the dopamine D2 receptor inhibitor is a dopamine D2 receptor antagonist.
(10) The pharmaceutical composition of (9), wherein the dopamine D2 receptor antagonist is selected from the group consisting of chlorpromazine, metoclopramide, domperidone, levomepromazine, fluphenazine, perphenazine, prochlorperazine, propericyazine, haloperidol, pipamperone, bromperidol, droperidol, quetiapine, asenapine, sulpiride, sultopride, tiapride, risperidone, mosapramine, zotepine, paliperidone, clocapramine, spiperone, nemonapride, timiperone, and perospirone.
(11) The pharmaceutical composition of (8), wherein the dopamine D2 receptor inhibitor is a dopamine D2 receptor-negative allosteric modulator.
(12) The pharmaceutical composition of (11), wherein the dopamine D2 receptor-negative allosteric modulator is SB269652.
(13) The pharmaceutical composition of (7), wherein the dopamine D1 and D2 receptor agonist is selected from the group consisting of levodopa, levodopa/carbidopa hydrate which is a levodopa combined formulation, levodopa/carbidopa/entacapone combination formulation, levodopa/benserazide hydrochloride combination formulation, and pergolide and rotigotine which are dopamine agonists.
(14) The pharmaceutical composition of (13), wherein levodopa and chlorpromazine are administered in combination.
(15) The pharmaceutical composition of any one of (7) to (14), characterized in that levodopa is orally administered in an amount at equal to or less than half the standard maintenance dose for Parkinson's disease treatment.
(16) The pharmaceutical composition of (15), wherein the amount of levodopa at equal to or less than half the standard maintenance dose for Parkinson's disease treatment is between 50 and 300 mg per day.
(17) The pharmaceutical composition of (16), wherein the amount of levodopa at equal to or less than half the standard maintenance dose for Parkinson's disease treatment is 50 mg per day.
(18) A pharmaceutical composition for treating obsessive-compulsive disorder comprising a dopamine D2 receptor inhibitor, wherein the composition is administered in combination with a dopamine D1 and D2 receptor agonist.
(19) The pharmaceutical composition of (18), wherein the dopamine D2 receptor inhibitor is selected from the group consisting of dopamine D2 receptor antagonists and dopamine D2 receptor-negative allosteric modulators.
(20) The pharmaceutical composition of (19), wherein the dopamine D2 receptor inhibitor is a dopamine D2 receptor antagonist.
(21) The pharmaceutical composition of (20), wherein the dopamine D2 receptor antagonist is selected from the group consisting of chlorpromazine, metoclopramide, domperidone, levomepromazine, fluphenazine, perphenazine, prochlorperazine, propericyazine, haloperidol, pipamperone, bromperidol, droperidol, quetiapine, asenapine, sulpiride, sultopride, tiapride, risperidone, mosapramine, zotepine, paliperidone, clocapramine, spiperone, nemonapride, timiperone, and perospirone.
(22) The pharmaceutical composition of (19), wherein the dopamine D2 receptor inhibitor is a dopamine D2 receptor-negative allosteric modulator.
(23) The pharmaceutical composition of (22), wherein the dopamine D2 receptor-negative allosteric modulator is SB269652.
(24) The pharmaceutical composition of (19), wherein the dopamine D1 and D2 receptor agonist is selected from the group consisting of levodopa, levodopa/carbidopa hydrate which is a levodopa combined formulation, levodopa/carbidopa/entacapone combination formulation, levodopa/benserazide hydrochloride combination formulation, and pergolide and rotigotine which are dopamine agonists.
(25) The pharmaceutical composition of (24), wherein chlorpromazine and levodopa are administered in combination.
(26) The pharmaceutical composition of (25), characterized in that chlorpromazine is orally administered in an amount at equal to or less than half the dose for anti-psychosis treatment as chlorpromazine hydrochloride.
(27) The pharmaceutical composition of (26), wherein the amount at equal to or less than half the dose for anti-psychosis treatment as chlorpromazine hydrochloride is between 5 to 30 mg per day.
(28) The pharmaceutical composition of (26), wherein the amount at equal to or less than half the dose for anti-psychosis treatment as chlorpromazine hydrochloride is 5 mg per day.
(29) A pharmaceutical composition for treating obsessive-compulsive disorder comprising a dopamine D1 receptor stimulator, wherein the composition is administered in combination with a dopamine D2 receptor inhibitor.
(30) The pharmaceutical composition of (29), wherein the dopamine D1 receptor stimulator is selected from the group consisting of dopamine D1 receptor agonists and dopamine D1 receptor-positive allosteric modulators, wherein the dopamine D2 receptor inhibitor is selected from the group consisting of dopamine D2 receptor antagonists and dopamine D2 receptor-negative allosteric modulators.

### EFFECT OF THE INVENTION

The present invention can provide highly effective and safe pharmaceutical products for obsessive-compulsive disorder.

### BRIEF DESCRIPTION OF THE DRAWINGS

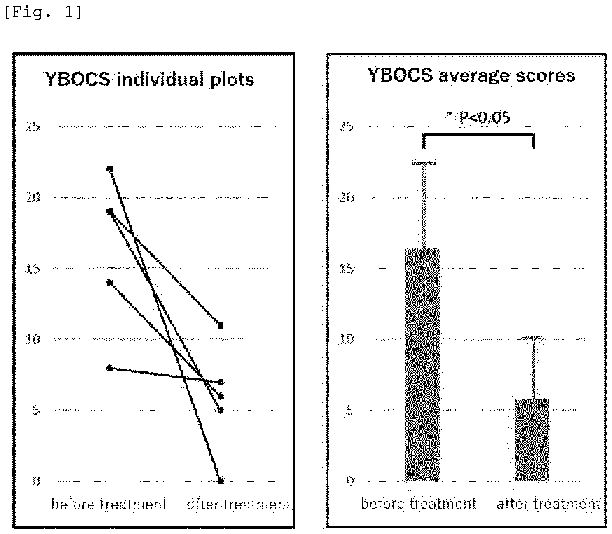

[Fig. 1] Figure 1 (left) is a graph of individual plots of measurement values for five patients with obsessive-compulsive disorder evaluated using YBOCS (self-reported YALE-BROWN Obsessive-Compulsive Scale). CPZ (Wintermin Fine Granules) and LDOPA (DOPACOL TABLETS) were used as therapeutic drugs. Figure 1 (right) is a graph showing the YBOCS average scores of five patients with obsessive-compulsive disorder before and after treatment. *, P < 0.05 (t-test)

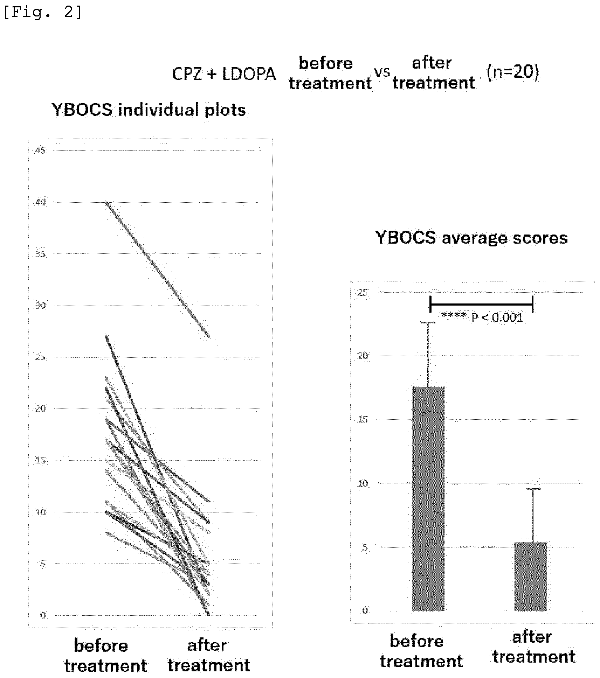

[Fig. 2] Figure 2 (left) is a graph of individual plots of measurement values for 20 patients with obsessive-compulsive disorder evaluated using YBOCS (self-reported YALE-BROWN Obsessive-Compulsive Scale). Figure 2 (right) is a graph showing the YBOCS average scores of 20 patients with obsessive-compulsive disorder before and after treatment. ****, P <0.001 (t-test). CPZ (Wintermin Fine Granules) and LDOPA (DOPACOL TABLETS) were used as therapeutic drugs.

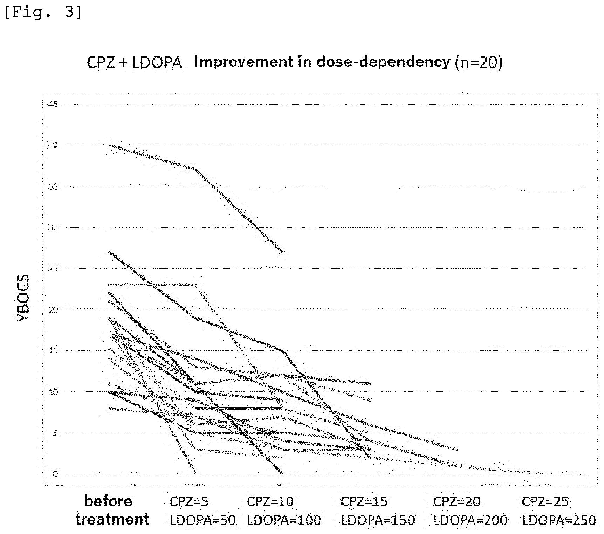

[Fig. 3] Figure 3 is plots showing the relationship between the YBOCS (self-reported YALE-BROWN Obsessive-Compulsive Scale) and the oral doses in the 20 patients with obsessive-compulsive disorder as indicated in Figure 2. The therapeutic effects were evaluated by gradually increasing the oral doses of the therapeutic drugs CPZ (Wintermin Fine Granules) and LDOPA (DOPACOL TABLETS).

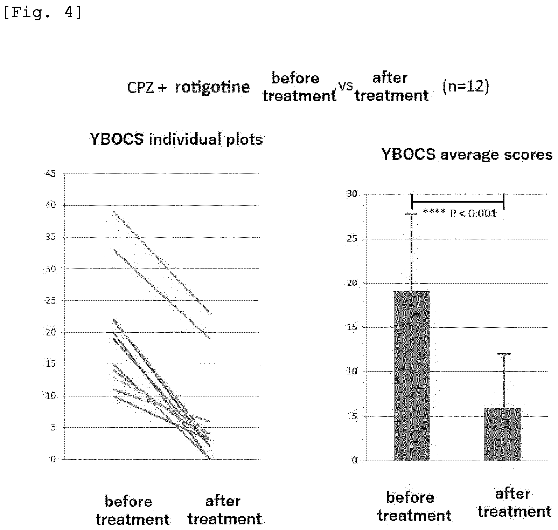

[Fig. 4] Figure 4 (left) is a graph of individual plots of measurement values for 12 patients with obsessive-compulsive disorder evaluated using YBOCS (self-reported YALE-BROWN Obsessive-Compulsive Scale). Figure 4 (right) is a graph showing the YBOCS average scores of 12 patients with obsessive-compulsive disorder before and after treatment. ****, P <0.001 (t-test). CPZ (Wintermin Fine Granules) and rotigotine (rotigotine transdermal patch: Newpropatch) were used as a therapeutic drug.

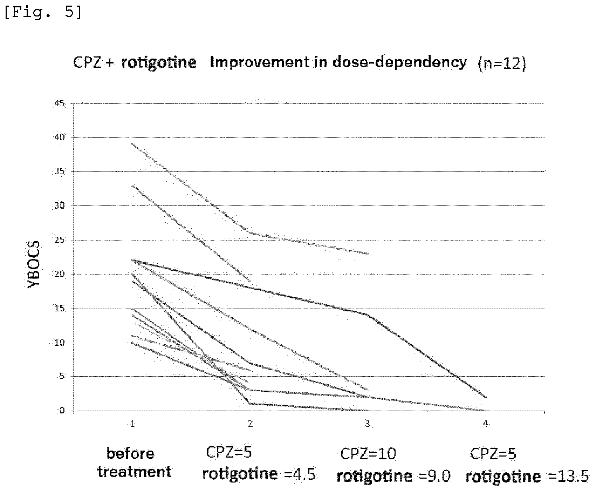

[Fig. 5] Figure 5 is plots showing the relationship between the YBOCS (self-administered YALE-BROWN Obsessive Compulsive Scale) and the oral and transdermal doses in the 12 patients with obsessive-compulsive disorder as indicated in Figure 4. The therapeutic effects were evaluated by gradually increasing the oral and transdermal doses of the therapeutic drugs CPZ (Wintermin Fine Granules) and rotigotine (rotigotine transdermal patch: Newpropatch).

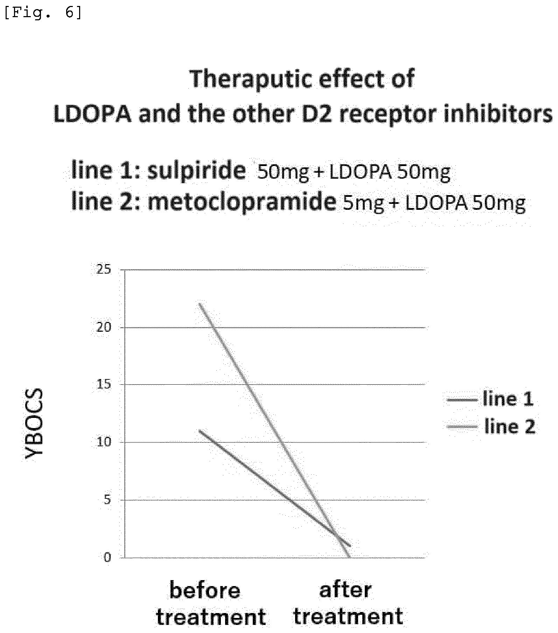

[Fig. 6] Figure 6 is a graph of individual plots of measurement values for two patients with obsessive-compulsive disorder evaluated using YBOCS (self-reported YALE-BROWN Obsessive-Compulsive Scale). The two individuals in Figure 6 were treated with dopamine D2 receptor antagonists other than CPZ (Wintermin Fine Granules) as therapeutic drugs. In lines 1 and 2, sulpiride (Dogmatil tablet) and metoclopramide (Primperan tablet) were respectively used instead of CPZ in combination with LDOPA (DOPACOL TABLET).

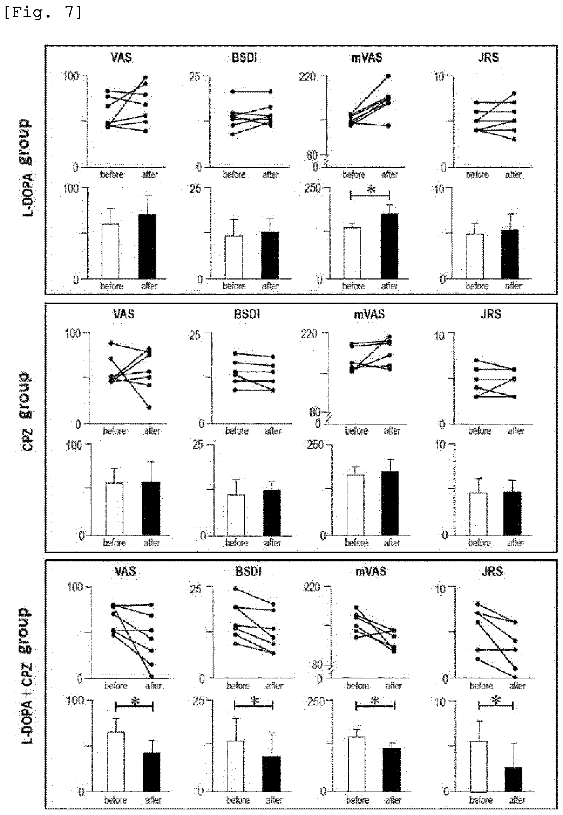

[Fig. 7] Figure 7 shows the measurement results of the severity of blepharospasm before and after a drug challenge test using Visual Analog Scale (VAS), Blepharospasm Disability Index (BSDI), modified VAS (mVAS), and Jancovic Rating Scale (JRS). Before: before the drug challenge test, After: after the drug challenge test.

In all of the L-DOPA group (top), the CPZ group (middle), and the L-DOPA + CPZ group (bottom), the number of patients is 7. The upper ones are polygonal line graphs showing the plots of the values for all cases, and the lower ones are graphs showing the average values for all cases. *, P <0.05 (Wilcoxon's signed-rank test).

### EMBODIMENTS FOR CARRYING OUT THE INVENTION

### <Pharmaceutical compositions>

In one embodiment, the present invention relates to a pharmaceutical composition for treating obsessive-compulsive disorder, which comprises a dopamine D1 receptor stimulator. The present inventors discovered the treatment of dystonia with LDOPA + CPZ as a past achievement, as shown in the last part of the specification. From the past report that has reported that dystonia frequently coexists with obsessive-compulsive disorder and depression, the present inventors inferred that LDOPA + CPZ would exert the same therapeutic effect for the latter diseases, and actually demonstrated for the first time that such therapeutic drugs can treat obsessive-compulsive disorder.

The articles previously reported include the followings, but none of them would lead to the present invention:
(1) Psychiatric disorders in adult-onset focal dystonia: a case-control study. Fabbrini G, Berardelli I, Moretti G, Pasquini M, Bloise M, Colosimo C, Biondi M, Berardelli A. Mov Disord. 2010 Mar 15; 5(4):459-65. doi: 10. 1002/mds. 22983.

The article shows that 51 of 89 patients with idiopathic dystonia (accounting for 57.3%) have some concomitant symptoms such as depression, anxiety disorders, and obsessive-compulsive disorder.

(2) Obsessive compulsive disorder among idiopathic focal dystonia patients: an epidemiological and family study. Cavallaro R, Galardi G, Cavallini MC, Henin M, Amodio S, Bellodi L, Comi G. Biol Psychiatry. 2002 Aug 15; 52(4):356-61. doi: 10. 1016/s0006-3223(02)01332-x.

The article reports that 19.7% of the patients with focal dystonia have concomitant obsessive-compulsive disorder.

(3) Psychiatric symptoms associated with focal hand dystonia. Voon V, Butler TR, Ekanayake V, Gallea C, Ameli R, Murphy DL, Hallett M. Mov Disord. 2010 Oct 15; 25(13): 2249-52. doi: 10. 002/mds. 23250.

The article reports that 12.82% of the patients with focal hand dystonia have concomitant obsessive-compulsive disorder, whereas 17.95% of them have concomitant depressive symptoms.

Such previous researches raise concerns that dysfunction of the striosome compartment of the striatum would involve the mechanism of expression of stereotyped symptoms, which are common elements in dystonia and obsessive-compulsive disorder. In this regard, several articles have been published, which hypothetically suggest that dysfunction in striosome compartment would be responsible for diseases with concurrent stereotyped symptoms, such as dystonia and obsessive-compulsive disorder (Professor Dr. Ann Graybiel).

(4) Graybiel AM, Canales JJ, Capper-Loup C. Levodopa-induced dyskinesias and dopamine-dependent stereotypies: a new hypothesis. Trends Neurosci. (2000) 23: S71-S77. doi: 10.1016/s1471-1931(00)00027-6

The article suggests that dysfunction of striosome compartment would induce dopamine overload in the matrix compartment to develop dystonia or obsessive-compulsive disorder with concurrent stereotyped symptoms.

(5) Crittenden JR, Graybiel AM. Basal ganglia disorders associated with imbalances in the striosome and matrix compartments. Front Neuroanat. (2011) 5; 59. doi: 10.3389/fnana.2011.00059

The article summarizes and discusses functional abnormalities (imbalance) in the striosome and matrix compartments are associated with multiple basal ganglia disorders such as Parkinson's disease, Huntington's disease, dystonia, and obsessive-compulsive disorder.

(6) Amemori KI, Gibb LG, Graybiel AM. Shifting responsibly: the importance of striatal modularity to reinforcement learning in uncertain environments. Front Hum Neurosci. (2011) 5:47. doi: 10.3389/fnhum.2011.00047

The article hypothetically indicates that imbalances between striosome and matrix compartments would cause dystonia, chorea, obsessive-compulsive disorder, depression, and other disorders by computer simulation, on the assumption that striatum is responsible for the module Reinforcement Learning (RL) based on stereotyped symptoms.

However, every one of the articles as described above is nothing more than a hypothesis, and no article describes any specific description regarding the treatment for diseases in human.

The previous researches or even the articles hypothetically suggesting its possibility do not describe any finding discovered by the present inventors, which a dopamine D1 receptor stimulator can improve dysfunction of striosomes. Regarding "dysfunction of striosomes", researchers are currently divided as to whether the dysfunction of the striosomes in dystonia and obsessive-compulsive disorder is due to hypofunction or a hyperfunction.

### (7) Japanese Publication No. 2015-52117

The article describes that the combination of a PDE7 inhibitor and a dopamine receptor agonist reduces preference and impulsivity towards drugs in a rodent model of drug addiction (cocaine or nicotine addiction).

First, it is important to note that there is any significant difference in the distribution pattern of D1/D2 receptors between the striatum of rodents and humans, from a comparative anatomical perspective, taking account of the comparison between the rodent drug addiction model according to the article and human obsessive-compulsive disorder according to the present invention. In basal ganglia disorders involving dopamine receptors, the therapeutic effects as observed in rodent animal models are unlikely to be directly recreated in human diseases.

The present inventors have published the following article, which details this issue: Specificity of striatal dopamine D1 system in humans: implications for clinical use of D1 receptor-agonists in Parkinson's disease. Goto S. Front Hum Neurosci. 2023 Apr 26;17:1178616. doi: 10.3389/fnhum.2023.1178616. eCollection 2023.

"Pharmacotherapy for obsessive-compulsive disorder targeting dopamine D1 signal in striosome system" is to utilize the human specificity of dopamine D1 system to normalize striosome D1 signal. However, the article describes that dopamine D1 receptors in the human striatum are selectively and dominantly present in the striosome compartment, whereas, in rodents, dopamine D1 receptors are uniformly distributed in both matrix and striosome compartments, and no bias towards the striosome compartment is observed.

Further, the strength of "preference and impulsivity towards drugs" observed in rodent models of drug addiction cannot be directly regarded as "strength of obsession, compulsive thoughts or compulsive behaviors in general daily life" in human obsessive-compulsive disorder. In DSM-5, Diagnostic and Statistical Manual of Mental Disorders according to the American Psychiatric Association, drug addiction is classified as "substance-related and addictive disorders", and is defined under a heading separate from "obsessive-compulsive and related disorders/obsessive-compulsive disorder and related disorders". Therefore, it cannot be predicted that the improvement of addiction to addictive substances in rodent models of drug addiction would directly lead to an improvement in the severity score of obsessive-compulsive disorder (YBOCS)in human.

### (8) CHEN, Fu-Feng et al., Brain Research, 2020, Vol. 1749, #147136, and PONNUSAMY, Ravikumar et al., Learning & Memory, 2005, Vol. 12, No. 4, p. 399-406

The former article describes that the administration of a D1 receptor agonist (SKF83959) enables to induce a long-term loss of fear memory in a rodent model using fear conditioning. The latter article describes that a single injection of a D2 receptor antagonist, sulpiride, was effective in erasing fear memory in a rodent model using fear conditioning as well.

In DSM-4, Diagnostic and Statistical Manual of Mental Disorders according to the American Psychiatric Association, obsessive-compulsive disorder was classified as one of the anxiety disorders. However, in the newer one, DSM-5, obsessive-compulsive disorder is no longer classified as the category of anxiety disorders, but rather as the category of "Obsessive-Compulsive and Related Disorders/Obsessive-Compulsive Disorder and Related Disorders". Therefore, "anxiety disorder" and "obsessive-compulsive disorder" are considered to be distinct disease groups as of now. In other words, anxiety disorder and obsessive-compulsive disorder should not be identified as the same disease. This means that the extinction of fear conditioning in animal models is not used as an explicit model for "anxiety disorders including obsessive-compulsive disorder". In other words, it is difficult to straightforwardly predict that the extinction of fear conditioning in a rodent model is used to improve the severity score of obsessive-compulsive disorder (YBOCS) in human, likely in article (7).

### (9) Elia Mota et al. Br J Pharmacol. 2021 Dec;178(24):4873-4890. doi: 10. 1111/bph.15664. Epub 2021 Oct 4

The article describes the results of the study about the influence of PDE10a on dopamine D1 and D2 cells in the striatum. However, the article describes no mention of obsessive-compulsive disorder.

According to the present invention, dopamine D1 receptor stimulator is selected from the group consisting of dopamine D1 receptor agonists and dopamine D1 receptor-positive allosteric modulators.

According to the present invention, the term "dopamine D1 receptor agonist" means a substance that stimulates the D1-like receptor family (D1 receptor and D5 receptor), which is responsible for excitability, among the five types of dopamine receptors including D1, D2, D3, D4, and D5 receptors, and it is also referred to as a D1 receptor stimulator. Therefore, "dopamine D1 receptor agonist" according to the present invention does not mean any specific agonist for dopamine D1 receptor, and does not exclude any substance stimulating other receptors such as D5 receptor in the D1-like receptor family.

Specific examples of dopamine D1 receptor agonists include SKF81297, SKF38393, SKF83959, SKF82526 (Fenoldopam), Dihydrexidine, ABT-431, A-86929, A-77636, A-68930, PF-06649751 (Tavapandone), PF-06412, and the like drugs (Jones-Tabah J, et al. The Signaling and Pharmacology of the Dopamine D1 Receptor. Front Cell Neurosci. 2022.). As of now, there are no approved drugs that have been authorized via clinical trials in human as D1 receptor agonists.

According to the present invention, the term "dopamine D1 receptor-positive allosteric modulator" means a substance that binds to the allosteric site of the dopamine D1 receptor to promote the agonist activity of the D1 receptor, which can be expected to have an effect equivalent to that of a dopamine D1 receptor agonist. This is abbreviated as D1-PAM. Regarding Allosteric Modulators against receptors, a ligand that binds to the allosteric site to promote the agonist activity of endogenous agonists is referred to as Positive Allosteric Modulators (PAM), whereas an allosteric ligand that inhibits the agonist activity is referred to as Negative Allosteric Modulators (NAM).

Specific examples of a dopamine D1 receptor-positive allosteric modulator include,
(1) DETQ (2-(2,6-dichlorophenyl)-1-((1S,3R)-3-(hydroxymethyl)-5-(2-hydroxypropan-2-yl)-1-methyl-3,4-dihydro-isoquinolin-2(1H)-yl)ethan-1-one) (Neuropharmacology 128 (2018) 351e365; Advances in Pharmacology Volume 86, 2019, Pages 273-305; Psychopharmacology (2023) 240:1033-1048);
(2) Mevidalen; LY3154207) (Cell 184, 943-956, February 18, 2021; Clinical Pharmacology in Drug Development 2022, 11(3) 324-332);
(3) MLS1082 (Mol Pharmacol 94:1197-1209, October 2018; Bioorg Med Chem Lett. 2021 January 01; 31);
(4) MLS6585 (Mol Pharmacol 94: 1197-1209, October 2018; Bioorg Med Chem Lett. 2021 January 01; 31);
(5) Pirazolyl-dihydroisoquinoline (ACS Med. Chem. Lett.)2020, 11, 4-4);
(6) DPTQ (Psychopharmacology (2023) 240:1033-1048);
(7) CID 2886111 ([N-(6-tert-butyl-3-carbamoyl-4,5,6,7-tetrahydro-1-benzothiophen-2-yl)pyridine-4-carboxamide]) (Mol Pharmacol 94:1232-1245, October 2018);
(8) LY3154885 (J. Med. Chem. 2022, 65, 3786-3797); and
(9) ASP4345 (Neuropsychopharmacology (2021) 46:1145-1151). Currently, there are no approved drugs that have been authorized via clinical trials in human as a D1 receptor-positive allosteric modulator.

In one embodiment, the present invention relates to a pharmaceutical composition for treating obsessive-compulsive disorder, which comprises a "dopamine D1 and D2 receptor agonist" or a "dopamine D2 receptor inhibitor", wherein an activity of a dopamine D1 receptor stimulator is obtained by administering a combination of a "dopamine D1 and D2 receptor agonist", which is usually administered for the treatment of diseases such as Parkinson's disease, and a "dopamine D2 receptor inhibitor", which is usually administered for the treatment of psychiatric disorders.

In one embodiment, the present invention relates to a pharmaceutical composition for treating obsessive-compulsive disorder, (1) which comprises a dopamine D1 receptor stimulator and/or (2) which comprises a dopamine D1 and D2 receptor agonist, wherein the pharmaceutical composition is administered in combination with a dopamine D2 receptor inhibitor, and/or (3) which comprises a dopamine D2 receptor inhibitor, wherein the pharmaceutical composition is administered in combination with a dopamine D1 and D2 receptor agonist.

According to the present invention, "administered in combination with" means administration modalities wherein a dopamine D1 and D2 receptor agonist and a dopamine D2 receptor inhibitor are administered, for example, simultaneously, with time intervals, or alternately. Therefore, when a dopamine D1 and D2 receptor agonist, and a dopamine D2 receptor inhibitor are simultaneously administered, they may be administered in the form of the same pharmaceutical composition, or they may be separately co-administered in the forms of the different pharmaceutical compositions. A dopamine D1 and D2 receptor agonist, and a dopamine D2 receptor inhibitor, may be administered separately with time intervals of seconds, minutes, hours, days, or weeks. Furthermore, a dopamine D1 and D2 receptor agonist, and a dopamine D2 receptor inhibitor, may be administered before or after other medications.

According to the present invention, a dopamine D1 and D2 receptor agonist, or a dopamine D2 receptor inhibitor may be administered in the same administration route, or may be administered in different administration route. For example, both compounds can be administered orally, or one compound can be administered orally while the other can be administered locally.

According to the present invention, the term "dopamine D1 receptor stimulator" is selected from the group consisting of dopamine D2 receptor antagonists and dopamine D2 receptor-negative allosteric modulators.

According to the present invention, the term "dopamine D2 receptor antagonist" means a substance that inhibits the D2-like receptor family (D2 receptor, D3 receptor, and D4 receptor), which is responsible for inhibition, among the five types of dopamine receptors including D1, D2, D3, D4, and D5 receptors, and it is also referred to as dopamine D2 receptor blocker or dopamine D2 receptor inhibitor. Therefore, the term "dopamine D2 receptor antagonist" according to the present invention does not mean any specific antagonist for dopamine D2 receptor, and means any antagonist as long as it inhibits or blocks dopamine D2 receptor. The term does not exclude any substance inhibiting or blocking other receptors such as D3 and D4 receptors in the D2-like receptor family.

Examples of dopamine D2 receptor antagonists include chlorpromazine, metoclopramide, domperidone, levomepromazine, fluphenazine, perphenazine, prochlorperazine, propericyazine, haloperidol, pipamperone, bromperidol, droperidol, quetiapine, asenapine, sulpiride, sultopride, tiapride, risperidone, mosapramine, zotepine, paliperidone, clocapramine, spiperone, nemonapride, timiperone, and perospirone.

According to the present invention, the term "dopamine D2 receptor-negative allosteric modulator" means a substance that binds to the allosteric site of the dopamine D2 receptor to inhibit the agonist activity of the D2 receptor, which can be expected to have an effect equivalent to that of a dopamine D2 receptor agonist. This is abbreviated as the D2-NAM. The dopamine D2 receptor-negative allosteric modulator is reviewed in Molecules 2023, 28, 178. https://doi.org/10.3390/molecules28010178.

Specific examples of the dopamine D2 receptor negative allosteric modulator include SB269652 (Mol Pharmacol 91:586-594, June 2017; J. Med. Chem. 2015, 58, 6819-6843; Biomedicines 2022, 10, 22. https://doi.org/10.3390/biomedicines10010022; J. Med. Chem. 2019, 62, 174-206). Currently, there are no approved drugs that have been authorized via clinical trials in human as a D2 receptor-negative allosteric modulator.

Examples of dopamine D1 and D2 receptor agonists include levodopa, levodopa/carbidopa hydrate which is a levodopa combined formulation, levodopa/carbidopa/entacapone combination formulation, levodopa/benserazide hydrochloride combination formulation, and pergolide and rotigotine which are dopamine agonists.

Levodopa is also referred to as L-3,4-dihydroxyphenylalanine (L-DOPA or LDOPA), and a full agonist for D1 and D2 receptors (D1R and D2R), which is the direct precursor of dopamine.

Levodopa, used as a dopamine D1 and D2 receptor agonist, is broken down by levodopa decarboxylase in the peripheral system (before crossing into the brain) and cannot enter the brain. Therefore, levodopa formulations contain carbidopa or benserazide, which suppresses the metabolism of levodopa in the peripheral system. An example of this is the combination formulation of levodopa and carbidopa (mainly marketed as "Neodopaston" and "Menesit") or the combination formulation of levodopa and benserazide (mainly marketed as "EC Dopal", "Neodopazol", and "Madopar"). According to the present invention, any combination formulation of them can be used as a dopamine D1 and D2 receptor agonist, and preferably, DOPACOL TABLETS are used. In case that DOPACOL TABLETS is administered according to the present specification, since the substantial pharmacological effect thereof is derived from levodopa, the administration of DOPACOL TABLETS can be considered to be essentially equivalent to the administration of levodopa.

In one embodiment, the present invention relates to a pharmaceutical composition for treating obsessive-compulsive disorder comprising a dopamine D1 receptor stimulator, wherein the composition is administered in combination with a dopamine D2 receptor inhibitor. It is expected that the combination of a dopamine D1 receptor stimulator with a dopamine D2 receptor inhibitor would have a greater effect in treating obsessive-compulsive disorder than the dopamine D1 receptor stimulator alone. Stimulation of the D1 receptor alone may increase the activity of the D2 receptor through secondary effects. Here, the definitions of the dopamine D1 receptor stimulator and the dopamine D2 receptor inhibitor are as described above.

As used herein, the term "treatment" or "treating" means a method or process aimed at (1) slowing down or halting the progression, aggravation or exacerbation of the symptoms of a disease or condition (e. g. obsessive-compulsive disorder); (2) inducing remission of the symptoms of a disease or condition; or (3) facilitating the cure of a disease or condition.

In one embodiment, the present invention relates to a pharmaceutical composition for treating obsessive-compulsive disorder comprising levodopa, characterized in that levodopa is orally administered in an amount at equal to or less than half the standard maintenance dose for Parkinson's disease treatment, and that simultaneously, chlorpromazine is orally administered in an amount at equal to or less than half the dose for anti-psychosis treatment as chlorpromazine hydrochloride, or metoclopramide is orally administered in an amount at equal to or less than half the dose for antiemetic treatment.

The standard maintenance dose for Parkinson's disease treatment as levodopa dosage is 200 to 250 mg per dose, and three times per day, which is a total of 600 to 750 mg per day. The dose for anti-psychosis treatment as chlorpromazine hydrochloride is 30 to 100 mg per day. According to the present invention, it is suitable that a pharmaceutical composition comprises a "dopamine D1 and D2 receptor agonist" wherein the amount at equal to or less than half the standard maintenance dose for Parkinson's disease treatment as levodopa ranges from 50 to 300 mg per day, preferably from 150 to 300 mg per day, and that a pharmaceutical composition comprises a "dopamine D2 receptor inhibitor" wherein the amount at equal to or less than half the dose for the antipsychotic treatment as chlorpromazine hydrochloride ranges from 5 to 30 mg per day, preferably from 5 to 30 mg per day. According to the present invention, it has been demonstrated that even a pharmaceutical composition comprising a "dopamine D1 and D2 receptor agonist" wherein the amount at equal to or less than half the standard maintenance dose for Parkinson's disease treatment as levodopa is 50 mg per day, and a pharmaceutical composition comprising a "dopamine D2 receptor inhibitor" wherein the amount at equal to or less than half the dose for the antipsychotic treatment as chlorpromazine hydrochloride is 5 mg per day, also have the effect of the present invention.

According to the present invention, a "pharmaceutical composition" comprises an effective amount of a "dopamine D1 and D2 receptor agonist" or a "dopamine D2 receptor inhibitor", and at least one pharmaceutically acceptable carrier or excipient. The term "effective amount" as used herein means any amount of a compound or composition that is sufficient to meet the intended purpose, such as the amount satisfying the desired biological or pharmacological response in cells, tissues, systems, or subjects. For example, the purpose according to a particular embodiment of the present invention is to slow down, alleviate or halt the progression, aggravation of exacerbation of obsessive-compulsive disorder, to induce remission of the symptoms of the disease, and/or to treat obsessive-compulsive disorder.

"Pharmaceutically acceptable carrier or excipient" can be formulated with an active ingredient and administered to a subject in need thereof via any route suitable for administration at a desired dosage. Various delivery systems are well known, which includes, for example, tablets, capsules, sustained-release formulations, injectables, encapsulated formulations in liposomes, powders, patches, microcapsules, and nanoparticle formulations.

The treatment according to the present invention can involve a single dose or multiple doses. Thus, administrations of a "dopamine D1 receptor stimulator", a "dopamine D1 and D2 receptor agonist", a "dopamine D2 receptor inhibitor", or a pharmaceutical composition comprising the same, may be carried out constantly or periodically over a specific period of time, and at specific intervals, for example, once every few hours, once a day, once a week, or once a month (e.g., time-release form).

In another embodiment, the present invention relates to a method for treating obsessive-compulsive disorder, which comprises administering a dopamine D1 receptor stimulator to a subject in need of such treatment. Furthermore, the present invention relates to a method for treating obsessive-compulsive disorder, which comprises administering a dopamine D1 and D2 receptor agonist in combination with a dopamine D2 receptor inhibitor to a subject in need of such treatment, and a method for treating obsessive-compulsive disorder, which comprises administering a dopamine D2 receptor inhibitor in combination with a dopamine D1 and D2 receptor agonist to a subject in need of such treatment.

Furthermore, the present invention relates to a dopamine D1 receptor stimulator for treating obsessive-compulsive disorder. Further, the present invention relates to a combination of a dopamine D1 and D2 receptor agonist and a dopamine D2 receptor inhibitor for treating obsessive-compulsive disorder.

In further another embodiment, the present invention relates to use of a dopamine D1 receptor stimulator for manufacturing a medicament for treating obsessive-compulsive disorder. The present invention relates to use of a combination of a dopamine D1 and D2 receptor agonist and a dopamine D2 receptor inhibitor for manufacturing a medicament for treating obsessive-compulsive disorder.

Hereinafter, the present invention will be described in detail by way of the reference examples and the working examples, but it should be noted that these would not limit the scope of the present invention and merely illustrate the invention.

### Examples

### Example 1

### Drug Challenge Test 1

Self-reported Yale-Brown Obsessive-Compulsive Scale (YBOCS) is a questionnaire designed to evaluate the severity of obsessive-compulsive symptoms in patients with obsessive-compulsive disorder for the purpose of assessing the effectiveness of pharmacotherapy (Non-patent Document 5). We used this evaluation scale to evaluate the symptoms.

The relationship between YBOCS and the severity of obsessive-compulsive disorder (OCD) is determined as follows (Storch EA, De Nadai AS, Conceicao do Rosario M et al Defining severity in adults with obsessive-compulsive disorder. Compr Psychiatry 63:30-35, 2015).

**Table 1**

| YBOCS score | : | severity of OCD |
|---|---|---|
| 35 to 40 | : | severe |
| 26 to 34 | : | moderate to severe |
| 14 to 25 | : | moderate |
| 0 to 13 | : | slight |

### Subjects

In this study, five patients with obsessive-compulsive disorder, aged 33 to 74 years old (one male and four females) were enrolled. The diagnosis of obsessive-compulsive disorder was made when the YBOCS score was 8 or higher (Non-patent Document 6).

The severity of obsessive-compulsive disorder was evaluated using YBOCS (Non-patent Documents 6, 7).

### Drugs

DOPACOL TABLETS L50 (registered trademark) (L-DOPA 50 mg + Carbidopa 5 mg, Nihon Igaku Kogyo Co., Ltd., Toyama, Japan) and Wintermin Fine Granules (10%) (registered trademark) (180 mg per 1 g of Chlorpromazine Phenol phthalate, Shionogi & Co., Ltd., Osaka, Japan) were used as drugs. Hereinafter, DOPACOL TABLETS L50 may be referred to as "DOPACOL", and Wintermin Fine Granules (10%) may be referred to as "CPZ".

### Dosing Regimen for CPZ

Regarding case numbers 1 to 4: The dosing regimen was to administer DOPACOL (1 tablet (50 mg)/day) + CPZ (5 mg)/day for the first 4 weeks, DOPACOL (1 tablet x 2 (100 mg)/day) + CPZ (5 mg) x 2/day for the next 4 weeks, and DOPACOL (1 tablet x 3 (150 mg)/day) + CPZ (5mg) x 3/day for the last 4 weeks.

Regarding case number 5: The following are the case when Metoclopramide tablet (Metoclopramide hydrochloride 5 mg, Takeda Pharmaceutical Co., Ltd.) was used.

A 66-year-old female developed symptoms at the age of 55. Due to concerns about her illness, she has visited multiple medical institutions and even been hospitalized. The YBOCS score was 22, and she was diagnosed as moderate obsessive-compulsive disorder. Oral medication of 50 mg of Levodopa and 5 mg of Metoclopramide was administered, and after 8 weeks, the symptoms completely disappeared (YBOCS: 0 points) .

The results are shown in Table 2 and Figure 1. The dosing regimen as described above improved the symptoms in all five cases with the initial introduction dose.

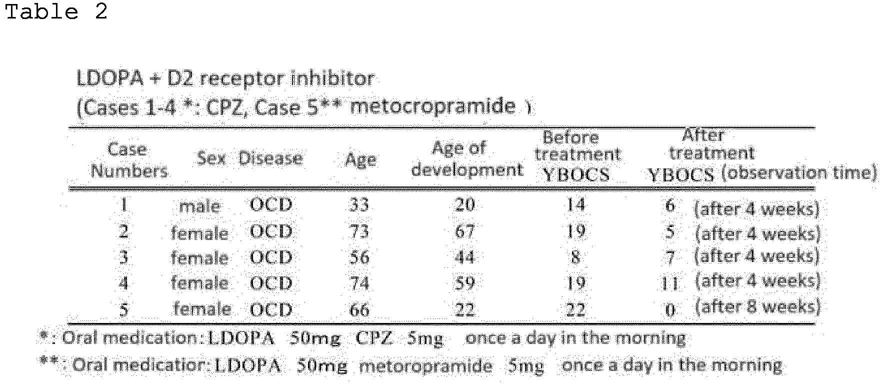

The drug administered in the test was DOPACOL TABLET, but its substantial pharmacological effect was derived from levodopa, and therefore the administration of levodopa (LDOPA) was described here.

As shown in Table 2 and Figure 1, improvement in symptoms was demonstrated in all cases who took small amounts of the dopamine D1 and D2 receptor agonist and the dopamine D2 receptor antagonists for a short period of time (average improvement rate of 65% on YBOCS, effective in all cases). It would be highly possible to accomplish the improvement by administering the higher doses for the longer period of time.

On the other hand, SSRI, which is currently considered to be the first choice for OCD treatment, is estimated effective if the improvement rate of YBOCS is 35%, but it has been reported that 40 to 61% of patients do not respond to SSRI (Pallanti S, Grassi G, Cantisani A. Emerging drugs to treat obsessive-compulsive disorder. Expert Opin Emerg Drugs. 2014;19(1):67-77. doi:10.1517/14728214.2014.875157; Pallanti S, Hollander E, Bienstock C, et al. Treatment non-response in OCD: methodological issues and operational definitions. Int J Neuropsychopharmacol. 2002;5(2):181-191. doi:10.1017/ S1461145702002900).

In addition, an additional dose of antipsychotics (D2 receptor antagonists) have been shown to be effective in OCD patients who are resistant to SSRI treatment, but even in that case, it has been reported that the effect is only about one-third of the total (Zhou DD, Zhou XX, Lv Z, et al. Comparative efficacy and tolerability of antipsychotics as augmentations in adults with treatment-resistant obsessive-compulsive disorder: a network meta-analysis. J Psychiatr Res. 2019;111:51-58. doi:10.1016/j. jpsychires.2019.01.014; Grassi G, Pallanti S. Current and up-and-coming pharmacotherapy for obsessive-compulsive disorder in adults. Expert Opin Pharmacother. 2018;19(14):1541-1550. doi:10.1080/14656566.2018.1528230).

### Example 2

### Effect of CPZ + LDOPA oral treatment on 20 patients with obsessive-compulsive disorder (OCD)

### Subjects

Twenty patients with obsessive-compulsive disorder, aged 18 to 82 years old (8 males and 12 females), were enrolled. The diagnosis of obsessive-compulsive disorder was made when the YBOCS score was 8 or higher (Non-patent Document 6).

The severity of obsessive-compulsive disorder was evaluated using the YBOCS (Non-patent Documents 6, 7). As shown in Table 3 and Figure 3, the clinical trial was continued with gradual increase in the oral dose for both. Note: The four subjects with case numbers 1 to 4 in Example 1 were included in the 20 subjects in Example 2. In detail, case numbers 4, 1, 2, and 3 in Example 1 (Table 2) correspond to the subjects of case numbers 1, 2, 4, and 6 in Example 2 (Table 3).

### Drugs

DOPACOL TABLETS L50 (registered trademark) (L-DOPA 50 mg + Carbidopa 5 mg, Nihon Igaku Kogyo Co., Ltd., Toyama, Japan) and Wintermin Fine Granules (10%) (registered trademark) (180 mg per 1 g of Chlorpromazine Phenol phthalate, Shionogi & Co., Ltd., Osaka, Japan) were used as drugs. Hereinafter, DOPACOL TABLETS L50 may be referred to as "DOPACOL", and Wintermin Fine Granules (10%) may be referred to as "CPZ".

### Dosing Regimen

The dosing regimen was conducted in accordance with Example 1, except for that the period until the next dose increase (dose escalation interval) in each subject was individually set ranging from every 2 weeks to every 12 weeks on the basis of the status of hospital visit and medication compliance of the subject (Table 3). The dose escalation intervals were fixed at every two weeks for the subjects who were able to visit the clinic every two weeks, and at every three months for the subjects who were able to visit the clinic every three months. In other words, the interval between dose increases in the same patient remained constant regardless of how many times it was increased. The initial dose was set at DOPACOL (1 tablet (50 mg)/day) + CPZ (5 mg)/day, the next dose administered at a fixed interval of at least 2 weeks was set at DOPACOL (1 tablet x 2 (100 mg)/day) + CPZ (5 mg) x 2 (10 mg)/day, and the subsequent dose was at DOPACOL (1 tablet x 3 (150 mg)/day) + CPZ (5 mg) x 3 (15 mg) /day. To further verify the dose-dependent effect, for two subjects (case numbers 5 and 10), the maximum dose was set to DOPACOL (1 tablet x 4 (200 mg)/day) + CPZ (5 mg) x 4 (20mg)/day at the third increase [each drug: 2 tablets in the morning, 1 tablet in the afternoon, and 1 tablet in the evening], and for one subject (case number 4), the maximum dose was set to DOPACOL (1 tablet x 5 (250 mg)/day) + CPZ (5 mg) x 5 (25 mg)/day at the fourth increase [each drug: 2 tablets in the morning, 2 tablets in the afternoon, and 1 tablet in the evening].

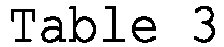

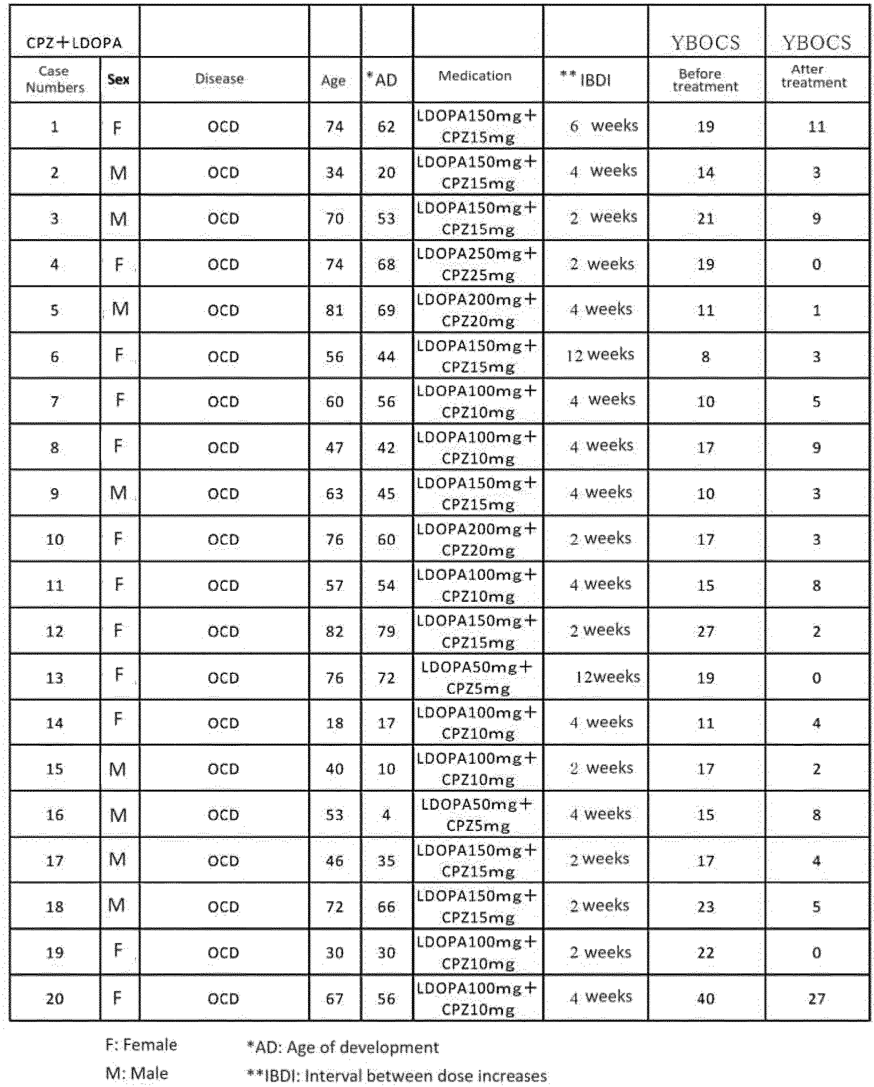

The drug administered in the test was DOPACOL TABLET, but its substantial pharmacological effect was derived from levodopa, and therefore the administration of levodopa (LDOPA) was described here.

The results are shown in Table 3 and Figure 2, which show the therapeutic effect in 20 patients with obsessive-compulsive disorder (OCD) who received the oral treatment with CPZ + LDOPA. According to Dosing Regimen as described above, improvement in symptoms was observed in all 20 cases (average improvement rate of 70% on YBOCS, effective in all cases). A t-test on the average YBOCS values before and after treatment detected a highly statistically significant difference of P = 0.000000004 (****P < 0.001).

Further, Figure 3 demonstrates the dose-dependent improvement effect on 20 patients with obsessive-compulsive disorder (OCD) who received the oral treatment with CPZ + LDOPA. In all 20 cases, gradual increase in dosage improved the OCD symptoms (YBOCS) in a dose-dependent manner. Symptom improvement was observed in a dose-dependent manner up to a maximum of CPZ = 25 mg/day and LDOPA = 250 mg/day, and in the subject of case number 4, the symptoms completely disappeared (YBOCS = 0) in the evaluation 10 weeks after administration.

### Example 3

### Effect of oral CPZ and transdermal rotigotine treatment on 12 patients with obsessive-compulsive disorder (OCD) Subjects

Twelve patients with obsessive-compulsive disorder, aged 24 to 82 years old (5 males and 7 females) were enrolled. The diagnosis of obsessive-compulsive disorder was made when the YBOCS score was 8 or higher (Non-patent Document 6). The severity of obsessive-compulsive disorder was evaluated using the YBOCS (Non-patent Documents 6, 7).

### Drugs

Newpropatch (registered trademark) (rotigotine 4.5 mg, 9 mg, and 13.5 mg per 1 patch of Newpropatch 4.5 mg, 9 mg, and 13.5mg, Otsuka Pharmaceutical Co., Ltd. Tokyo, Japan), Wintermin Fine Granules (10%) (registered trademark) (180mg per 1 g of chlorpromazine phenol maleate, Shionogi & Co., Ltd. Osaka, Japan) were used. Hereinafter, Newpropatch may be referred to as "NP", and Wintermin Fine Granules (10%) may be referred to as "CPZ". Newpropatch is a transdermal patch of rotigotine, which is a type of dopamine receptor agonists.

### Dosing Regimen

The dosing regimen was conducted in accordance with Example 1, except for that the period until the next dose increase (dose escalation interval) in each subject was individually set ranging from every 2 weeks to every 6 weeks on the basis of the status of hospital visit and medication compliance of the subject (Table 4). Newpropatch was applied to any normal skin on the shoulder, upper arm, abdomen, flank, buttocks, or thigh, and was replaced every 24 hours. CPZ was administered orally in accordance with Example 1. The initial dose was set at Newpropatch 4.5 mg/day + CPZ (5 mg)/day, the next dose administered at a fixed interval of at least 2 weeks was set as Newpropatch 9 mg/day + CPZ (5mg) x 2 (10 mg)/day, and the maximum dose was set at Newpropatch 13.5 mg/day + CPZ (5mg) x 3 (15 mg)/day.

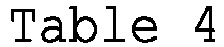

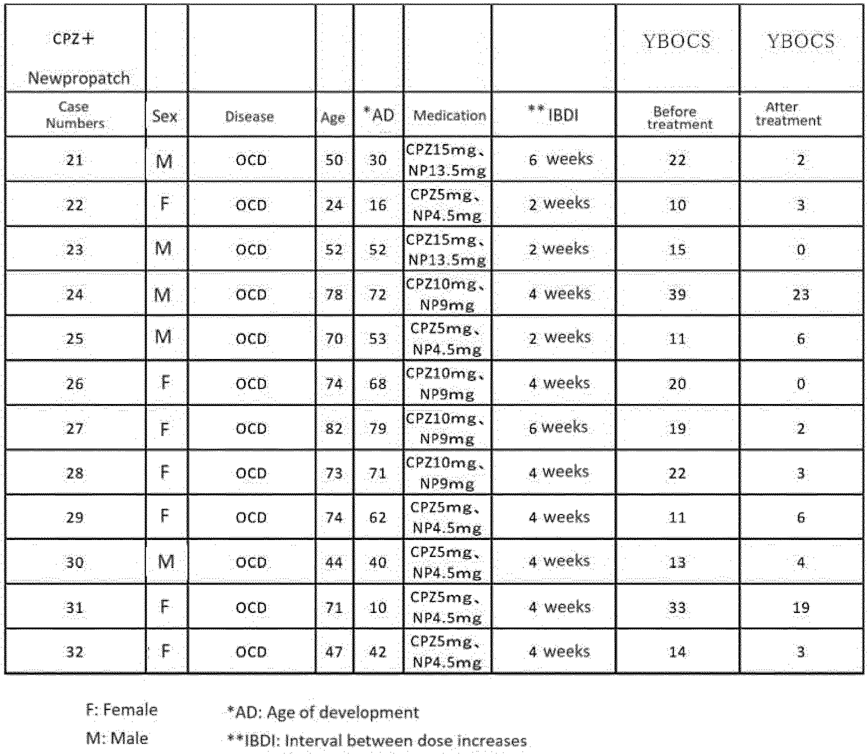

The results are shown in Table 4 and Figure 4, which show the therapeutic effects in 12 patients with obsessive-compulsive disorder (OCD) treated with the therapy of oral administration of CPZ + transdermal application of rotigotine. According to Dosing Regimen as described above, improvement in symptoms was observed in all 12 cases (average improvement rate of 69% on YBOCS, effective in all cases). A t-test on the average YBOCS values before and after treatment detected a highly statistically significant difference of P = 0000006 (****P < 0.001).

Further, Figure 5 demonstrates the dose-dependent improvement effect on 12 patients with obsessive-compulsive disorder (OCD) who received the treatment with the therapy of oral administration of CPZ + transdermal application of rotigotine. In all 12 cases, gradual increase in dosage improved the OCD symptoms (YBOCS) in a dose-dependent manner. In two subjects of case numbers 23 and 26, the symptoms completely disappeared (YBOCS=0) in the evaluations 10 weeks after administrated (CPZ = 15, NP = 13.5) and 8 weeks after administrated (CPZ = 10, NP = 9), respectively.

### Example 4

### Effect of oral treatment of D2 receptor antagonists other than CPZ + LDOPA on two patients with obsessive-compulsive disorder (OCD)

Substantially according to the method of Example 1, the therapeutic effects of metoclopramide and sulpiride were investigated as other D2 receptor antagonists except for CPZ.

The following are the cases when using metoclopramide tablet (Metoclopramide hydrochloride 5mg, Takeda Pharmaceutical Co., Ltd.).
A 66-year-old female patient (case number 5 in Example 1): Onset at age 55. Due to concerns about her illness, she has visited multiple medical institutions and even been hospitalized. The YBOCS score was 22, and she was diagnosed as moderate obsessive-compulsive disorder. Oral medication of 50 mg of Levodopa and 5 mg of Metoclopramide was administered, and after 8 weeks, the symptoms completely disappeared (YBOCS: 0 points). From the CPZ administration group in Figure 1, the results of case number 5 with metoclopramide administration were plotted in Figure 6. The drug administered in the test was DOPACOL TABLET, but its substantial pharmacological effect was derived from levodopa, and therefore the administration of levodopa (LDOPA) was described here.

The following are the case when using Dogmatil tablets (50 mg sulpiride, NichiIko Pharmaceutical Co., Ltd., Toyama, Japan). A 55-year-old male. Onset at age 37. He experienced compulsive behaviors such as checking for failures, rewriting, and rereading, and his life was painful. The YBOCS score was 11, and he was diagnosed as mild obsessive-compulsive disorder. Fifty mg of L-DOPA and 50 mg of sulpiride were orally administered once a day in the morning, and YBOCS was evaluated after 4 weeks. The drug administered in the test was DOPACOL TABLET, but its substantial pharmacological effect was derived from levodopa, and therefore the administration of levodopa (LDOPA) was described here.

The results are shown in Figure 6, which shows the improvement effect of OCD symptoms (YBOCS) in two patients with obsessive-compulsive disorder (OCD) treated with D2 receptor antagonists other than CPZ and LDOPA. In both one case who received metoclopramide and another case who received sulpiride, instead of CPZ, significant improvement in symptoms was observed at the evaluation after 4 weeks with a low initial dose (sulpiride 50m g + LDOPA 50 mg, metoclopramide 5 mg + LDOPA 50 mg). In line 2 using metoclopramide, symptoms completely disappeared (YBOCS=0), and in line 1 using sulpiride, symptoms almost disappeared (YBOCS=1). These results suggest that the combination of LDOPA and D2 receptor antagonists other than CPZ provides a sufficiently effective clinical effect.

The present inventor has also found a pharmaceutical composition comprising levodopa for treating dystonia based on the facts of the following reference example as one aspect. The pharmaceutical composition is characterized in that levodopa is orally administered in an amount at equal to or less than half the standard maintenance dose for Parkinson's disease treatment, and at the same time, chlorpromazine is orally administered in an amount at equal to or less than half the dose for anti-psychotic treatment as chlorpromazine hydrochloride. Hereinafter, the fact is described.

### Reference Example 1

### Summary

For the first time, the present inventors demonstrated that the dual dopamine modification therapy using L-3,4-dihydroxyphenylalanine (L-DOPA; levodopa) and chlorpromazine (CPZ) showed therapeutic effects on a type of focal dystonia, blepharospasm. L-DOPA is a direct precursor of dopamine and a full agonist for D1 and D2 receptors (D1R and D2R), while CPZ is an effective antagonist for D2R.

### Subjects

In this study, 21 patients with blepharospasm aged 51 to 79 years old (6 males and 15 females) were enrolled, with an average age of 68.7 ± 7.8 years old). The diagnosis of blepharospasm was conducted according to the Fahn's definition (1. Fahn S., Marsden CD. and Calne DB. "Classification and Investigation of Dystonia". In: Marsden CD and Fahn S, editors., Movement Disorders, Butterworths, London, (1987). p. 332-58.).

To exclude hereditary and secondary dystonia, clinical and genetic testing was performed along with brain magnetic resonance imaging (brain MRI). In genetic testing, the pathogenic variants of known dystonia genes were screened by the whole exome sequencing (OMIM Phenotypic Series PS128100). All patients who received the Botulinum Toxin Type A (BTX-A) treatment were instructed to have a washout period of three months from their last BTX-A injection to the start of this clinical trial. Video analysis was conducted before and after the drug challenge test. The severity of blepharospasm was evaluated using four indicators: Visual Analog Scale (VAS) [reference 12], Blepharospasm Disability Index (BSDI) [13], the Modified VAS [14], and Jankovic Rating Scale (JRS) [13, 15].

### Drugs

DOPACOL TABLETS L50 (registered trademark) (L-DOPA 50 mg + Carbidopa 5 mg, Nihon Igaku Kogyo Co., Ltd., Toyama, Japan) and Wintermin Fine Granules (10%) (registered trademark) (180 mg per 1 g of Chlorpromazine Phenol phthalate, Shionogi & Co., Ltd., Osaka, Japan) were used as drugs. Hereinafter, DOPACOL TABLETS L50 may be referred to as "DOPACOL", and Wintermin Fine Granules (10%) may be referred to as "CPZ".

### Patient Sorting and Drug Administration

The patients were randomly divided into three groups (L-DOPA group, CPZ group, and L-DOPA+CPZ group) in order of arrival at the hospital. For a double-blind method, participants and evaluators were not informed of the identification of each group.
L-DOPA group: This group included seven patients with blepharospasm (three males and four females) who received DOPACOL alone. The age range was from 51 to 79 years old with an average age of 66.0 ± 10.1 years old) and the average duration of illness was 10.7 ± 9.5 years. For the first two weeks, DOPACOL (1 tablet/day) was administered, for the following two weeks, DOPACOL (1 tablet x 2/day) were administered, and for the last four weeks, DOPACOL (1 tablet x 3/day) were administered.
CPZ group: This group included seven patients with blepharospasm (one male and six females) who received CPZ alone. The age range was 60-72 years old with an average age of 66.4 ± 3.9 years) and the average duration of illness was 8.4 ± 5.9 years. For the first two weeks, CPZ (5 mg/day) was administered, for the following two weeks, CPZ (5 mg x 2/day) were administered, and for the last four weeks, CPZ (5 mg x 3/day) were administered.
L-DOPA+CPZ group: This group included seven patients with blepharospasm (two males and five females) who received both DOPACOL and CPZ. The age range was 66 to 78 years old with an average age of 73.5 ± 5.9 years and the average duration of illness was 8.0 ± 7.6 years. For the first 2 weeks, DOPACOL (1 tablet/day) and CPZ (5 mg/day) were administered, for the following 2 weeks, DOPACOL (1 tablet x2/day) and CPZ (5 mg x 2/day) were administered, and for the last 4 weeks, DOPACOL (1 tablet x3/day) and CPZ (5 mg x 3/day) were administered.

### Statistical analysis

The statistical significance was evaluated using the Wilcoxon signed-rank test or the Analysis of Variance for one factor (ANOVA). The statistical significance was set at P <0.05. The statistical analysis was conducted using the SPSS statistical software (version 11.0 for Windows) (IBM Corp., Armonk, New York, USA).

### Results:

The severity of blepharospasm was evaluated using VAS and BSDI as subjective symptoms, and using mVAS and JRS as objective symptoms (Figure 7). First, there was no significant difference in the severity of subjective and objective symptoms among all groups of L-DOPA, CPZ, and L-DOPA+CPZ before the drug challenge test (P> 0.05, one-way ANOVA). In other words, it was confirmed that there was no significant difference in the severity before the drug challenge test among the three groups.

Next, the severities before and after the drug challenge test were compared for each symptom. L-DOPA group (Figure 7; top):
In the corrected VAS [Before the drug challenge test: 141.3 ± 8.7 (n=7), After the drug challenge test, 178.8 ± 24.3 (n=7) "Before" vs "After"; P <0.05, Wilcoxon signed-rank test], the severity of symptoms significantly progressed, whereas in VAS, BSDI and JRS, no severity progressed.

### CPZ group (Figure 7; center):

No significant changes in severity were observed before and after the drug challenge test for all assessments by VAS, BSDI, mVAS, and JRS.

### L-DOPA + CPZ group (Figure 7; below):

After administration of L-DOPA + CPZ, the severity of both subjective and objective symptoms in VAS, BSDI, mVAS, and JRS significantly decreased compared to before the drug challenge test. In VAS [Before drug challenge test 67.1 ± 14.6 (n=7), After drug challenge test 44.8 ± 27.5 (n=7), "Before" vs "After"; P <0.05, Wilcoxon signed-rank test], BSDI [Before drug challenge test 15.1 ± 6.6 (n=7), After drug challenge test 10.6 ± 6.7 (n=7), "Before" vs "After"; P <0.05, Wilcoxon signed-rank test], mVAS [Before drug challenge test 148.0 ± 17.4 (n=7); After drug challenge test 116.5 ± 14.5 (n=7), "Before" vs "After"; P <0.05, Wilcoxon signed-rank test], and JRS [Before drug challenge test 5.9 ± 2.2 (n=7), After drug challenge test 3.0 ± 2.3 (n=7), "Before" vs "After"; P <0.05, Wilcoxon signed-rank test].

No apparent neurological or neuropsychiatric changes were observed during the drug challenge test in all of L-DOPA, CPZ, and L-DOPA+CPZ groups. Therefore, it has been confirmed that the therapeutic effect for blepharospasm can only be achieved by the combination of DOPACOL (1 tablet × 3/day) and CPZ (5 mg × 3/day), rather than by the administration of DOPACOL alone or CPZ alone.

Dystonia is a pathological condition that causes abnormal posture and movement disorders due to sustained muscle contractions. Many of the pediatric cases are suffered from generalized dystonia with genetic mutations, whereas many of the adult cases are suffered from focal dystonia. Focal dystonia is common, and includes conditions such as blepharospasm, cervical dystonia, and writer's cramp. The present inventors discovered accidentally that the administration of sulpiride or metoclopramide in addition to levodopa, alleviated dystonia, and during World Congress of Neurology - Kyoto, held on September 16-21, 2017, presented this finding (World Congress of Neurology - Kyoto, held on September 16-21, 2017 in the abstract collection of the congress: VOLUME 381, SUPPLEMENT, 580, OCTOBER 15, 2017 Three cases of dystonia with effective levodopa and D2 blocker combination therapy S. Marsumoto, M.Takahashi, DOI:https://doi.org/10.1016/ j.jns.2017.08.1635).

Possible inventions according to the above aspect are described below:

### <Pharmaceutical compositions comprising levodopa for treating dystonia>

[1] A pharmaceutical composition comprising levodopa for treating dystonia, wherein the composition is characterized in that levodopa is orally administered in an amount at equal to or less than half the standard maintenance dose for Parkinson's disease treatment, and at the same time, chlorpromazine is orally administered in an amount at equal to or less than half the dose for anti-psychotic treatment as chlorpromazine hydrochloride; Specifically, the pharmaceutical composition wherein the amount as the levodopa dose at equal to or less than half the standard maintenance dose for Parkinson's disease treatment is at a dose of 50 to 300 mg per day, preferably 50 to 200 mg per day, more preferably 100 to 150 mg per day, and the pharmaceutical composition comprising levodopa for treating dystonia wherein the amount as chlorpromazine hydrochloride at a dose of less than half the antipsychotic treatment dose, is 10 to 15 mg per day: and

### <Pharmaceutical compositions comprising chlorpromazine for treating dystonia>

[2] A pharmaceutical composition comprising chlorpromazine for treating dystonia, wherein the composition is characterized in that chlorpromazine is orally administered in an amount at equal to or less than half the dose for anti-psychotic treatment as chlorpromazine hydrochloride, and at the same time, levodopa is orally administered in an amount at equal to or less than half the standard maintenance dose for Parkinson's disease treatment; Specifically, the pharmaceutical composition wherein the amount of chlorpromazine hydrochloride at equal to or less than half the dose for antipsychotic treatment is 5 to 300 mg per day, preferably 5 to 200 mg per day, more preferably 10 to 15 mg per day, and the pharmaceutical composition comprising chlorpromazine for treating dystonia, wherein the levodopa dose is less than half the standard maintenance dose for Parkinson's disease treatment is 100 to 150 mg per day.

As described above, the present invention may encompass the following embodiments:

### <Pharmaceutical compositions>

(101) A pharmaceutical composition for treating obsessive-compulsive disorder, which comprises a dopamine D1 receptor stimulator.

(102) The pharmaceutical composition of (101), wherein the dopamine D1 receptor stimulator is selected from the group consisting of dopamine D1 receptor agonists and dopamine D1 receptor-positive allosteric modulators.

(103) The pharmaceutical composition of (102), wherein the dopamine D1 receptor stimulator is a dopamine D1 receptor agonist.

(104) The pharmaceutical composition of (103), wherein the dopamine D1 receptor agonist is selected from the group consisting of SKF81297, SKF38393, SKF83959, SKF82526 (fenoldpam), dihydrexidine, ABT-431, A-86929, A-77636, A-68930, PF-06649751 (tavapandon), and PF-06412.

(105) The pharmaceutical composition of (102), wherein the dopamine D1 receptor stimulator is a dopamine D1 receptor-positive allosteric modulator.

(106) The pharmaceutical composition of (105), wherein the dopamine D1 receptor-positive allosteric modulator is selected from the group consisting of DETQ (2-(2,6-dichlorophenyl)-1-((1S, 3R)-3- (hydroxymethyl)-5-(2-hydroxypropan-2-yl)-1-methyl-3,4-dihydroisoquinolin-2(1H)-yl)ethan-1-one), mevidalen, MLS1082, MLS6585, pyrazolyldihydroisoquinoline, DPTQ, CID 2886111 ((N-(6-tert-butyl-3-carbamoyl-4,5,6,7-tetrahydro-1-benzothiophen-2-yl)pyridine-4-carboxamide), LY3154885, and ASP4345.

(107) A pharmaceutical composition for treating obsessive-compulsive disorder comprising a dopamine D1 and D2 receptor agonist, wherein the composition is administered in combination with a dopamine D2 receptor inhibitor.

(108) The pharmaceutical composition of (107), wherein the dopamine D2 receptor inhibitor is selected from the group consisting of dopamine D2 receptor antagonists and dopamine D2 receptor-negative allosteric modulators.

(109) The pharmaceutical composition of (108), wherein the dopamine D2 receptor inhibitor is a dopamine D2 receptor antagonist.

(110) The pharmaceutical composition of (109), wherein the dopamine D2 receptor antagonist is selected from the group consisting of chlorpromazine, metoclopramide, domperidone, levomepromazine, fluphenazine, perphenazine, prochlorperazine, propericyazine, haloperidol, pipamperone, bromperidol, droperidol, quetiapine, asenapine, sulpiride, sultopride, tiapride, risperidone, mosapramine, zotepine, paliperidone, clocapramine, spiperone, nemonapride, timiperone, and perospirone.

(111) The pharmaceutical composition of (108), wherein the dopamine D2 receptor inhibitor is a dopamine D2 receptor-negative allosteric modulator.

(112) The pharmaceutical composition of (111), wherein the dopamine D2 receptor-negative allosteric modulator is SB269652.

(113) The pharmaceutical composition of any one of (107) to (112), wherein the dopamine D1 and D2 receptor agonist is selected from the group consisting of levodopa, levodopa/carbidopa hydrate which is a levodopa combined formulation, levodopa/carbidopa/entacapone combination formulation, levodopa/benserazide hydrochloride combination formulation, and pergolide and rotigotine which are dopamine agonists.

(114) The pharmaceutical composition of (113), wherein levodopa and chlorpromazine are administered in combination.

(115) The pharmaceutical composition of any one of (107) to (114), characterized in that levodopa is orally administered in an amount at equal to or less than half the standard maintenance dose for Parkinson's disease treatment.

(116) The pharmaceutical composition of (115), wherein the amount of levodopa at equal to or less than half the standard maintenance dose for Parkinson's disease treatment is between 50 and 300 mg per day.

(117) The pharmaceutical composition of (115), wherein the amount of levodopa at equal to or less than half the standard maintenance dose for Parkinson's disease treatment is 50 mg per day.

(18)
A pharmaceutical composition for treating obsessive-compulsive disorder comprising a dopamine D2 receptor inhibitor, wherein the composition is administered in combination with a dopamine D1 and D2 receptor agonist.

(119) The pharmaceutical composition of (118), wherein the dopamine D2 receptor inhibitor is selected from the group consisting of dopamine D2 receptor antagonists and dopamine D2 receptor-negative allosteric modulators.

(120) The pharmaceutical composition of (119), wherein the dopamine D2 receptor inhibitor is a dopamine D2 receptor antagonist.

(121) The pharmaceutical composition of (120), wherein the dopamine D2 receptor antagonist is selected from the group consisting of chlorpromazine, metoclopramide, domperidone, levomepromazine, fluphenazine, perphenazine, prochlorperazine, propericyazine, haloperidol, pipamperone, bromperidol, droperidol, quetiapine, asenapine, sulpiride, sultopride, tiapride, risperidone, mosapramine, zotepine, paliperidone, clocapramine, spiperone, nemonapride, timiperone, and perospirone.

(122) The pharmaceutical composition of (119), wherein the dopamine D2 receptor inhibitor is a dopamine D2 receptor-negative allosteric modulator.

(123) The pharmaceutical composition of (122), wherein the dopamine D2 receptor-negative allosteric modulator is SB269652.

(124) The pharmaceutical composition of any one of (119) to (124), wherein the dopamine D1 and D2 receptor agonist is selected from the group consisting of levodopa, levodopa/carbidopa hydrate which is a levodopa combined formulation, levodopa/carbidopa/entacapone combination formulation, levodopa/benserazide hydrochloride combination formulation, and pergolide and rotigotine which are dopamine agonists.

(125) The pharmaceutical composition of (124), wherein chlorpromazine and levodopa are administered in combination.

(126) The pharmaceutical composition of (125), characterized in that chlorpromazine is orally administered in an amount at equal to or less than half the dose for anti-psychosis treatment as chlorpromazine hydrochloride.

(127) The pharmaceutical composition of (126), wherein the amount at equal to or less than half the dose for anti-psychosis treatment as chlorpromazine hydrochloride is between 5 to 30 mg per day.

(128) The pharmaceutical composition of (26), wherein the amount at equal to or less than half the dose for anti-psychosis treatment as chlorpromazine hydrochloride is 5 mg per day.

(129) A pharmaceutical composition for treating obsessive-compulsive disorder comprising a dopamine D1 receptor stimulator, wherein the composition is administered in combination with a dopamine D2 receptor inhibitor.

(130) The pharmaceutical composition of (129), wherein the dopamine D1 receptor stimulator is selected from the group consisting of dopamine D1 receptor agonists and dopamine D1 receptor-positive allosteric modulators, wherein the dopamine D2 receptor inhibitor is selected from the group consisting of dopamine D2 receptor antagonists and dopamine D2 receptor-negative allosteric modulators.

## Claims

1. A pharmaceutical composition for treating obsessive-compulsive disorder, which comprises a dopamine D1 receptor stimulator.

2. The pharmaceutical composition of claim 1, wherein the dopamine D1 receptor stimulator is selected from the group consisting of dopamine D1 receptor agonists and dopamine D1 receptor-positive allosteric modulators.

3. The pharmaceutical composition of claim 2, wherein the dopamine D1 receptor stimulator is a dopamine D1 receptor agonist.

4. The pharmaceutical composition of claim 3, wherein the dopamine D1 receptor agonist is selected from the group consisting of SKF81297, SKF38393, SKF83959, SKF82526 (fenoldpam), dihydrexidine, ABT-431, A-86929, A-77636, A-68930, PF-06649751 (tavapandon), and PF-06412.

5. The pharmaceutical composition of claim 2, wherein the dopamine D1 receptor stimulator is a dopamine D1 receptor-positive allosteric modulator.

6. The pharmaceutical composition of claim 5, wherein the dopamine D1 receptor-positive allosteric modulator is selected from the group consisting of DETQ (2-(2,6-dichlorophenyl)-1-((1S,3R)-3-(hydroxymethyl)-5-(2-hydroxypropan-2-yl)-1-methyl-3,4-dihydroisoquinolin-2(1H)-yl)ethan-1-one), mevidalen, MLS1082, MLS6585, pyrazolyldihydroisoquinoline, DPTQ, CID 2886111 ([N-(6-tert-butyl-3-carbamoyl-4,5,6,7-tetrahydro-1-benzothiophen-2-yl)pyridine-4-carboxamide), LY3154885, and ASP4345.

7. A pharmaceutical composition for treating obsessive-compulsive disorder comprising a dopamine D1 and D2 receptor agonist, wherein the composition is administered in combination with a dopamine D2 receptor inhibitor.

8. The pharmaceutical composition of claim 7, wherein the dopamine D2 receptor inhibitor is selected from the group consisting of dopamine D2 receptor antagonists and dopamine D2 receptor-negative allosteric modulators.

9. The pharmaceutical composition of claim 8, wherein the dopamine D2 receptor inhibitor is a dopamine D2 receptor antagonist.

10. The pharmaceutical composition of claim 9, wherein the dopamine D2 receptor antagonist is selected from the group consisting of chlorpromazine, metoclopramide, domperidone, levomepromazine, fluphenazine, perphenazine, prochlorperazine, propericyazine, haloperidol, pipamperone, bromperidol, droperidol, quetiapine, asenapine, sulpiride, sultopride, tiapride, risperidone, mosapramine, zotepine, paliperidone, clocapramine, spiperone, nemonapride, timiperone, and perospirone.

11. The pharmaceutical composition of claim 8, wherein the dopamine D2 receptor inhibitor is a dopamine D2 receptor-negative allosteric modulator.

12. The pharmaceutical composition of claim 11, wherein the dopamine D2 receptor-negative allosteric modulator is SB269652.

13. The pharmaceutical composition of claim 7, wherein the dopamine D1 and D2 receptor agonist is selected from the group consisting of levodopa, levodopa/carbidopa hydrate which is a levodopa combined formulation, levodopa/carbidopa/entacapone combination formulation, levodopa/benserazide hydrochloride combination formulation, and pergolide and rotigotine which are dopamine agonists.

14. The pharmaceutical composition of claim 13, wherein levodopa and chlorpromazine are administered in combination.

15. The pharmaceutical composition of any one of claims 7 to 14, **characterized in that** levodopa is orally administered in an amount at equal to or less than half the standard maintenance dose for Parkinson's disease treatment.

16. The pharmaceutical composition of claim 15, wherein the amount of levodopa at equal to or less than half the standard maintenance dose for Parkinson's disease treatment is between 50 and 300 mg per day.

17. The pharmaceutical composition of claim 15, wherein the amount of levodopa at equal to or less than half the standard maintenance dose for Parkinson's disease treatment is 50 mg per day.

18. A pharmaceutical composition for treating obsessive-compulsive disorder comprising a dopamine D2 receptor inhibitor, wherein the composition is administered in combination with a dopamine D1 and D2 receptor agonist.

19. The pharmaceutical composition of claim 18, wherein the dopamine D2 receptor inhibitor is selected from the group consisting of dopamine D2 receptor antagonists and dopamine D2 receptor-negative allosteric modulators.

20. The pharmaceutical composition of claim 19, wherein the dopamine D2 receptor inhibitor is a dopamine D2 receptor antagonist.

21. The pharmaceutical composition of claim 20, wherein the dopamine D2 receptor antagonist is selected from the group consisting of chlorpromazine, metoclopramide, domperidone, levomepromazine, fluphenazine, perphenazine, prochlorperazine, propericyazine, haloperidol, pipamperone, bromperidol, droperidol, quetiapine, asenapine, sulpiride, sultopride, tiapride, risperidone, mosapramine, zotepine, paliperidone, clocapramine, spiperone, nemonapride, timiperone, and perospirone.

22. The pharmaceutical composition of claim 19, wherein the dopamine D2 receptor inhibitor is dopamine D2 receptor-negative allosteric modulator.

23. The pharmaceutical composition of claim 22, wherein the dopamine D2 receptor-negative allosteric modulator is SB269652.

24. The pharmaceutical composition of claim 19, wherein the dopamine D1 and D2 receptor agonist is selected from the group consisting of levodopa, levodopa/carbidopa hydrate which is a levodopa combined formulation, levodopa/carbidopa/entacapone combination formulation, levodopa/benserazide hydrochloride combination formulation, and pergolide and rotigotine which are dopamine agonists.

25. The pharmaceutical composition of claim 24, wherein chlorpromazine and levodopa are administered in combination.

26. The pharmaceutical composition of claim 25, **characterized in that** chlorpromazine is orally administered in an amount at equal to or less than half the dose for anti-psychosis treatment as chlorpromazine hydrochloride.

27. The pharmaceutical composition of claim 26, wherein the amount at equal to or less than half the dose for anti-psychosis treatment as chlorpromazine hydrochloride is between 5 to 30 mg per day.

28. The pharmaceutical composition of claim 26, wherein the amount at equal to or less than half the dose for anti-psychosis treatment as chlorpromazine hydrochloride is 5 mg per day.

29. A pharmaceutical composition for treating obsessive-compulsive disorder comprising a dopamine D1 receptor stimulator, wherein the composition is administered in combination with a dopamine D2 receptor inhibitor.

30. The pharmaceutical composition of claim 29, wherein the dopamine D1 receptor stimulator is selected from the group consisting of dopamine D1 receptor agonists and dopamine D1 receptor-positive allosteric modulators, wherein the dopamine D2 receptor inhibitor is selected from the group consisting of dopamine D2 receptor antagonists and dopamine D2 receptor-negative allosteric modulators.
